# EUROPEAN PATENT SPECIFICATION

(11) **EP 3 634 318 B1**
(45) Date of publication and mention of the grant of the patent: **09.02.2022**
(21) Application number: 18730418.3
(22) Date of filing: 18.05.2018
(51) Int. Cl.: A61F 2/38, A61F 2/30, A61F 2/44

(54) **METHODS FOR MANUFACTURING IMPLANTABLE TISSUE REPAIR DEVICES**
VERFAHREN ZUR HERSTELLUNG IMPLANTIERBARE GEWEBEREPARATURVORRICHTUNGEN
PROCÉDÉS DE FABRICATION DES DISPOSITIFS DE RÉPARATION TISSULAIRE IMPLANTABLES

(30) Priority: 05.06.2017 GB 201708909
(43) Date of publication of application: 15.04.2020
(73) Proprietor: Orthox Limited, Abingdon, Oxfordshire OX14 4RQ (GB)
(72) Inventor: SKAER, Nicholas James Vavasour, Abingdon Oxfordshire OX14 4RQ (GB); WALKER, Robert Alan, Abingdon Oxfordshire OX14 4RQ (GB)
(74) Representative: Wilson Gunn
(86) International application number: PCT/GB2018/051349
(87) International publication number: WO 2018/224800

(56) References cited:
- WO-A1-2010/014446
- WO-A1-2010/151589
- US-A1- 2007 179 607
- US-A1- 2014 324 169

## Description

### Technical Field of the Invention

This invention relates to methods of manufacturing implantable tissue repair devices. In particular, the implantable tissue repair devices comprise solvated structural components such as hydrogels and which are adapted to be at least partly inserted into cavities or apertures within a tissue, or which are secured adjacent to said tissue.

### Background to the Invention

Cartilage in the adult mammalian body occurs in three principal forms: hyaline cartilage; white fibrocartilage; and yellow elastic cartilage. Hyaline cartilage is chiefly present as articular cartilage in the synovial diarthroidal joints e.g. the knee, hip and shoulder, and between long bones, where it forms the stiff and smooth articulating surfaces. White fibrocartilage is present in the menisci of the knee and temporomandibular joint of the jaw and in intervertebral discs. Yellow elastic cartilage gives support to the epiglottis, Eustachian tube and external ear.

Three pathological conditions involving cartilage damage are very common: osteoarthrosis of articular cartilage; injury to the fibrocartilage of the knee menisci; collapse, rupture or herniation of the intervertebral disc; and damage caused by rheumatoid arthritis. Osteoarthrosis is caused by the progressive damage and breakdown of articular cartilage commonly in the ankle, hip, shoulder and knee (and in many other joint types including elbow, fingers etc.) and is an important cause of pain and reduced mobility in young and old people alike. Injury to the fibrocartilage of the meniscus is a common sports injury and is also seen as a result of road traffic accidents and other traumatic injuries.

Articular cartilage is highly specialized to provide a relatively frictionless, highly lubricated, wear resistant surface between relatively rigid bones. It also functions to transmit and distribute the forces arising from loaded contact to the surrounding cartilage and underlying subchondral trabecular bone. It is a nonvascular connective tissue largely composed of a fluid phase consisting principally of water and electrolytes interspersed in a solid phase containing type II collagen fibrils, proteo-glycan and other glycoproteins. The latter constituents surround, and are secreted by, highly specialized mesenchymal cells, the chondrocytes, which account for some 10% of the volume of articular cartilage. The collagen fibrils within articular cartilage are arranged in a complex arcade structure forming columns arranged normal to and anchored in the osteochondral junction. These columns run up through the deep layer of cartilage, but the predominant fibre orientation gradually changes to form the arches of the arcade structure in the superficial cartilage. In the superficial layer which abuts the joint space, the meshwork of collagen fibrils is much denser while the fibrils are almost entirely tangential to the cartilage surface. The orientation of collagen in articular cartilage is vital to its mechanical function. Healthy articular cartilage is strong and stiff (modulus between 1 and 20 MPa).

No wholly satisfactory procedure exists for replacing damaged articular cartilage in osteoarthrosis and instead in the case of the two most frequently injured joints, the hip and knee, and also other joints such as elbows, shoulders, ankle and extremities, artificial prostheses are most commonly used to replace the entire joint. While these increase mobility and reduce pain they suffer from progressive wear, mechanical failure, adverse tissue reactions and loosening at their interphase with the bone. Accordingly, there has been much work around the area of providing a suitable implantable repair material with improved performance over the currently available prostheses.

One such device is described in WO 2007/020449 A2, describing a cartilaginous tissue repair device with a biocompatible, bioresorbable three-dimensional silk or other fibre lay and a biocompatible, bioresorbable substantially porous silk-based or other hydrogel partially or substantially filling the interstices of the fibre lay.

International patent application number PCT/IB2009/051775 (published under WO2009/133532 A2) discloses a silk fibroin solution and method that can be used to make an improved fibroin material that has been found to be efficient as an implant for cartilage repair. The method for the preparation of the regenerated silk fibroin solution comprises the steps of: (a) treating the silk or silk with an ionic reagent comprising aqueous solutions of one or more of ammonium hydroxide, ammonium chloride, ammonium bromide, ammonium nitrate, potassium hydroxide, potassium chloride, potassium bromide or potassium nitrate; (b) subsequently drying the silk or silk cocoons after treatment of the silk or silk cocoons with the ionic reagent; and (c) subsequently dissolving the silk or silk cocoons in a chaotropic agent.

Furthermore, International patent application number PCT/GB2009/050727 (published under WO2009/156760 A2) discloses method for the preparation of an implantable material for the repair, augmentation or replacement of bone from a fibroin solution. The method comprises: preparing a gel from fibroin solution; preparing a material by subjecting the gel to one or more steps of freezing and thawing the gel, wherein the step of preparing the gel from the fibroin solution is performed in the presence of phosphate ions. The material is generally treated with calcium ions to form a fibroin-apatite. A further method step comprises the step of treating the material with an isocyanate to form cross-links. The implantable material has been found to be efficient as an implant for bone repair.

WO2010151589 describes a trapezium prosthesis comprising a hydrogel. The prosthesis has a first dehydrated configuration and a second partially rehydrated configuration, the first configuration being of reduced size relative to the second configuration. In the first configuration the prosthesis may be dehydrated, e.g. by solvent exchange.

Whilst implantable cartilaginous tissue repair devices of the prior art are all useful in the repair, augmentation or replacement of damaged cartilage, many such devices suffer from a number of problems, such as: a) failure to anchor securely to existing bone or cartilage; b) failure to integrate with existing bone or cartilage; c) failure of the devices after implantation due to wrinkling, warping or shrinking of the devices over time, or through loosening of the device from its anchor; and d) failure of the device to maintain its overall shape under load within or on the repaired tissue.

In particular many devices suffer from difficulties in effectively and permanently securing the devices to bone, cartilage or other tissue. It is therefore an aim of embodiments of the present invention to provide an implantable repair device capable of load bearing and with improved or enhanced abilities to integrate with, or to be secured to, existing bone or cartilage. It is another aim of embodiments of the present invention to provide an implantable repair device adapted to provide improved articulation of the joint following cartilage replacement.

It would also be advantageous to provide an implantable tissue repair device with improved anchoring in apertures or cavities formed within bone or cartilage, and with improved resistance to any anchoring of the device being torn or detached from the device and/or the aperture or cavity within the bone or cartilage.

It would furthermore be advantageous to provide a device which is flexible under load but which is relatively resistant to permanent shape change, such as wrinkling, warping and shrinking, after fully implanting the device within or on damaged tissue.

It would also be advantageous to provide a device which overcomes or mitigates at least one problem of the prior art described herein.

### Summary of the Invention

According to a first aspect of the invention there is provided a method of manufacturing an implantable tissue repair device for the repair, replacement or augmentation of a biological tissue, the method comprising: providing a device comprising a biocompatible solvated structural material comprising one or more anchoring elements; and the method characterised by comprising the steps of compressing and drying at least the or each anchoring element to reduce one or more dimensions of at least part of the device.

The method may comprise retaining the device in the compressed and dried or dehydrated state until use.

The method may comprise securing the compressed part in the compressed state, for example by binding the compressed part in a biocompatible and biodegradable binding material. Such a material may comprise polylactide, polyglycolide or polylactide-polyglycolide material, for example.

Dehydrating or drying the structural material has the advantage that the structural material, in its solvated state, has already assumed the final shape of the part of the device and drying may shrink the part in the same overall shape and configuration. Subsequent rehydration will decompress or relax the hydrogel back to its original shape, thereby retaining the original (pre-compressed, pre-dried) architecture of the device.

In other embodiments, the method comprises compressing at least part of the device followed by freezing at least the compressed part. In such embodiments, the freezing of the part maintains the part in the compressed state.

In preferred embodiments, the method comprises firstly compressing at least a part of the device to reduce at least one dimension of the device, and subsequently drying at least the compressed part either during or after compression. This ensures that the part or parts of the device is first compressed to smaller dimensions, then dried to prevent decompression of the part or parts when the compression force on the device is removed, thereby eliminating the need for any outside means of maintaining the part or parts in the compressed state.

In particularly preferred embodiments the part or parts of the device (especially the anchoring elements) is/are compressed to reduce at least one dimension, and the compressed part is freeze-dried, to ensure that the part or parts maintains the original shape and configuration of the solvated, uncompressed part,; and which can then be re-solvated to relax/decompress or reswell the compressed part back to its original (or substantially original) dimensions.

In the dehydrated state, the part of the device, or the device *per se,* may be immersed or packaged in a solvent-free medium, in order to ensure the device remains in the dried state. The device may be stored in the solvent-free medium until just before use. The device may be packaged in a solvent-free gas, such as in nitrogen, for example.

The device may comprise a body and the device comprises one or more anchoring elements (arranged in use to anchor the device to bone, cartilage or other biological tissue) and the method may comprise compressing and drying at least part of the body and the anchoring element or elements. Compression and drying of the anchoring element or elements may comprise reducing one or more dimensions of the, or each, anchoring element, such as reducing the width, diameter, circumference and/or or perimeter thereof.

The compression and/or drying of at least part of the structural material is reversible and therefore, in use, the device may be inserted into an aperture, cavity or the like, in a tissue to be repaired, replaced or augmented such that the reduced dimension part may be easily located in the aperture, cavity or the like and then the compression and/or shrinkage of the part can be reversed by decompression and/or re-solvation; such that the dimension(s) of the part increase, substantially fill the aperture, cavity or the like and the part then tightly grips the aperture or cavity wall to provide a secure anchorage. The part inserted into the aperture or cavity may be configured such that decompression or re-solvation causes the part to expand or relax back to dimensions greater than the aperture or cavity, and due to the resilient nature of the structural material from which it is made, will resiliently grip the wall or walls of the aperture or cavity, thereby significantly decreasing the chance of the part being removed from the aperture or cavity in use.

The anchoring elements may comprise a projection extending from a main body of the device. The projection may comprise a tubular projection or a projection comprising any other suitable shape (such as a keel, wedge, ridge or the like, for example), arranged in use to be located in an aperture, slot or cavity in or adjacent to a tissue to be repaired, replaced or augmented. There may be two or more projections, arranged in use to be located in holes or cavities in or adjacent to a tissue.

In preferred embodiments, the or each anchoring element is compressed then dried, and compression may be maintained during drying.

The part or parts of the device which are compressed and/or dried may be shrunk to no more than 98%, 97%, 96%, 95%, 90%, 80%, 75%, 70%, 65%, 60%, 55% or no more than 50% of part's or parts' original (uncompressed or non-dehydrated) volume, width, diameter, circumference and/or perimeter, and optionally length/height. In some embodiments, the part (or parts) is shrunk to between 60% and 75% of its uncompressed or non-dried volume, dimensions, circumference, perimeter and/or height. Preferably there are two or more anchoring elements comprising tubular projections and each anchoring element is compressed and dried to shrink the element to between 60% and 75% of its original volume, dimensions, circumference or perimeter.

The method may comprise at least partially drying the whole device or the whole structural material of the device, such as the structural material forming the body and anchoring elements.

Drying of the part or whole of the device may comprise freeze drying the part or whole of the device. In order to ensure the structural material does not crack or deform during freeze-drying, the device or part thereof may be immersed or contacted with a lyo-protectant during freeze-drying to protect the part from stresses and replace part of the solvent lost through drying. Suitable lyo-protectants include saccharides such as trehalose and sucrose, polymers such as polyvinylpyrrolidone or polyvinyl alcohol, glycerol or other poly-ols, for example. Glycerol may be preferred due to its ability to soften the dried structural material during and after freeze drying. Treatment with a lyo-protectant, such as glycerol, may be performed before drying or freeze-drying, while the structural material is in its solvated state. The use of a lyo-protectant is particularly useful for hydrogel materials (as the structural material). The use of a lyo-protectant is also particularly useful for structural material, such as hydrogels, containing pores, especially containing an open porous network of inter-connected pores, as the lyo-protectant may penetrate the pores and provide lyo-protection from both within and without the device during drying or freeze-drying.

In preferred embodiments, the part of parts of the solvated structural material is compressed and frozen in the compressed state.

In particularly preferred embodiments, the method may comprise compressing the solvated structural material followed by drying the compressed structural material, especially compressing, freezing and drying, such as by compressing, freezing and freeze-drying. In some embodiments, the method comprises compressing part or the whole of the device followed by drying the part or whole of the device. In such embodiments, drying is preferably performed by freeze-drying as discussed and defined herein. Compression of the part or whole of the device may be as described hereinabove and the part or whole of the device is preferably shrunk to between 50% to 80% of the size of the solvated part or device. In preferred embodiments, the device comprises a body and a plurality of anchoring elements and at least the anchoring elements are compressed and dried. In some embodiments, substantially only the anchoring elements are compressed and dried. In preferred embodiments only the anchoring elements are compressed and the whole of the body and anchoring elements (or the whole device) is dried.

The structural material may comprise a material selected from silk fibroin, fibrin, fibronectin, cellulose, alginate, hyaluronic acid, gelatin and collagen. In preferred embodiments, the hydrogel comprises silk fibroin. The silk fibroin may be a regenerated silk fibroin and the silk fibroin may be regenerated mulberry, wild or spider silk fibroin.

The solvent of the solvated structural material may comprise water or an aqueous solvent. In such embodiments, all references to "drying" and "dried" may be considered to be dehydration or dehydrating. In other embodiments, the solvent may be another biocompatible solvent such as ethanol, for example; however, in preferred embodiments the solvent is water or an aqueous medium.

The method may comprise locating at least one fibre, or a network or layer of fibres at least partially within the structural material. The fibres or network or layer of fibres may be as described herein above. The network of fibres may comprise a two-dimensional or three-dimensional network of fibres. Suitable two dimensional networks or layers include a mesh, net or web. The network of fibres may include at least one fibre network layer. Suitable three-dimensional networks include a plurality of stacked fibre layers, for example.

The fibres or fibre network may be partially dissolved in the body of the device, such that an outer surface of the fibres substantially blends, melds or merges into the structural material. This forms a stronger, reinforced body, increasing the strength of the device.

The solvated structural material may be a hydrogel, which may be formed by gelling a hydrogel precursor solution. The hydrogel precursor solution may comprise a solution of monomer, dimers, oligomers or polymers in a suitable solvent. The fibres, fibre network or fibre layer may be located within the precursor solution before gelling of the solution.

In embodiments, wherein a part of the structural material is compressed and/or dehydrated, and the dimensions of the part or the whole of the device are reduced, the fibres may also compress or contract. The fibres may then decompress or expand upon decompression or rehydration of the hydrogel.

In embodiments, wherein the device comprises a body from which project a plurality of integrally formed anchoring elements and the body and anchoring elements comprise fibre layers, the fibre layer in the anchoring elements may be stitched to the fibre layer in the body via one or more threads. The threads may comprise any suitable biocompatible thread materials, such as polyester, nylon or the like for example, and may comprise suture threads.

The threads may be configured to bend or concertina within the structural material when the part or whole of the structural material is compressed and/or dehydrated and the part or whole of the structural material is shrunk. In this way the threads may maintain their relative positions within the device during compression and/or dehydration of the structural material.

The method may comprise locating a rigid support such as a sheet or framework within the structural material. The rigid support may be positioned within the structural material such that it is not exposed or does not protrude from the structural material when the part or whole of the structural material is compressed and/or dehydrated. The rigid support may therefore be set in from any outer surfaces of the structural material (or device) at a distance which ensures it is not exposed or does not protrude from the surfaces of the compression and/or dehydration of the structural material. The rigid support may be located within the structural material precursor solution before gelling.

The dried or dehydrated material may be adapted to substantially re-solvate or rehydrate over a time period of between 30 second and 60 minutes when placed in a solvent environment of tissue into or onto which the device has been implanted, and in some embodiments between 1 minute and 30 minutes or between 2 minutes and 10 minutes.

### Detailed Description of the Invention

In order that the invention may be more clearly understood one or more embodiments thereof will now be described, by way of example only, with reference to the accompanying drawings, of which:
Figure 1 illustrates a side cross-sectional view of an embodiment of a device made by the method of the invention with all parts in an uncompressed and hydrated state;
Figure 2 illustrates a side cross-sectional view of the embodiment of Figure 1 with the anchoring elements in a compressed and dehydrated state (with the equivalent uncompressed and hydrated dimensions shown in dotted lines);
Figure 3 illustrates a side view of a second embodiment of a device made by the method of the invention in a compressed and dehydrated state;
Figure 4A illustrates a side cross-sectional view of the device of Figures 1 and 2 in place in a lesion in cartilage, with the anchoring elements in a compressed and dehydrated state;
Figure 4B illustrates a side cross-sectional view of the device of Figure 1 and 2 in place in a lesion in cartilage, with the anchoring elements in a decompressed and rehydrated state;
Figure 5A illustrates the device of Figure 3 in place in a lesion in cartilage with the device in a compressed and dehydrated state;
Figure 5B illustrates the device of Figure 3 in place in a cartilage lesion, in a decompressed and rehydrated state;
Figure 6 illustrates a side sectional view through a third embodiment of a device made by the method of the invention with the anchoring elements in a decompressed and dehydrated state;
Figure 7A illustrates a perspective view of another embodiment of a device made by the method of the invention with the anchoring elements in a compressed and freeze-dried state, spanning a tear in gut wall; and
Figure 7B illustrates the device of Figure 7A in spanning the gut wall, with the anchoring elements in a decompressed and rehydrated state.

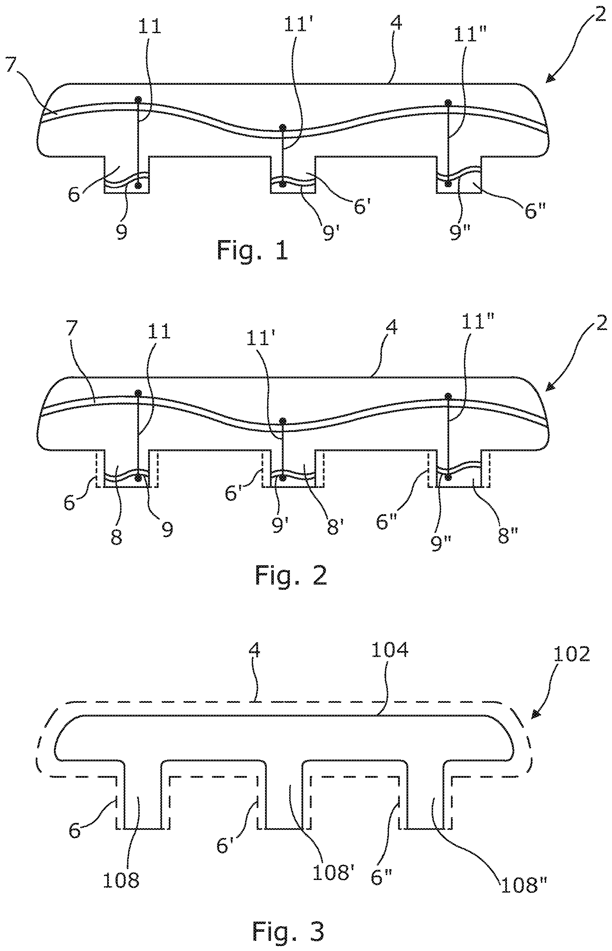

Figure 1 illustrates a side view of an implantable tissue repair device 2 made by the method of the invention. The device 2 includes a device body 4 and a number of anchoring elements 6, 6', 6". The anchoring elements 6, 6', 6" are integrally formed and project from the body 4. Both the body and the anchoring elements are formed from a structural material comprising a silk fibroin hydrogel which is both stiff and resilient. The device 4 is suitable as a device for the repair of articular cartilage; for example, it may be inserted into a tear, lesion or other cavity within damaged articular cartilage.

The body 4 includes a fibre layer comprising a silk fibre mesh 7 extending therethrough. The anchoring elements 6, 6',6" each include a corresponding fibre layer 9,9',9" extending therethrough. The fibre layer 7 of the body and the fibre layers 9,9',9" are connected via nylon sutures 11,11',11" stitched therebetween. The fibre layers 7,9,9',9" and sutures 11,11',11" serve to provide structural support and load bearing to the device 2.

The device 2 of Figure 1 is in a fully hydrated and uncompressed state. Figure 2 illustrates the same device 2 in which the anchoring elements 6, 6', 6" have been firstly compressed, and then dehydrated to maintain the anchoring elements 6. 6', 6" in a compressed state. As can be seen from Figure 2, the anchoring element 6, 6', 6" in their compressed and dehydrated state have shrunk such that both the diameter and volume of the anchoring element 6, 6', 6" are less than the fully hydrated and non-compressed state. The compressed and dehydrated anchoring elements are shown with reference numerals 8, 8', 8".

The device is processed as follows to achieve the configuration shown in Figure 2. After compression of the anchoring elements 8, 8′, 8‴ to reduce their diameter and volume, the whole device 2, including the body 4 undergoes freezing and then freeze-drying. The compression force may then be removed, and the anchoring elements 8, 8′, 8ʺ are maintained with reduced dimensions due to the freeze-drying dehydration. Importantly the overall shape of the device is maintained after freeze-drying, with only the anchoring elements having reduced dimensions but also having the same shape as the uncompressed and hydrated anchoring elements.

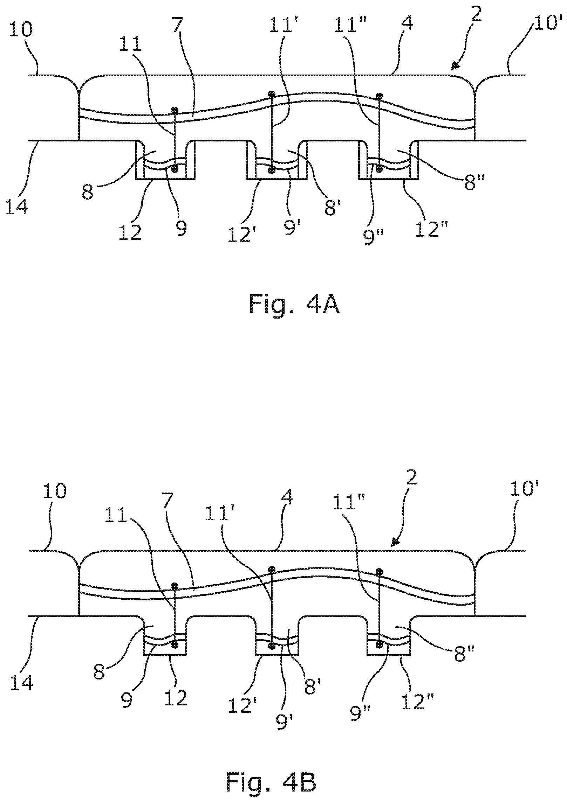

Use of the device 2 of Figure 2 will now be described with reference to Figures 4A and 4B. The device 2 is inserted into a cavity formed into articular cartilage. The articular cartilage is connected to a bone surface 14. The articular cartilage has a cavity bounded by an articular cartilage wall 10, 10', as shown in Figure 1. The bone surface 14 is prepared by a practitioner drilling cavities 12, 12', 12" into the bone surface 14, as shown in Figure 4A. In this configuration, the articular cartilage wall 10, 10' bounds a cavity through which the bone surface 14 is accessible, and in which the holes 12, 12', 12" are also accessible. In use the body 4 of the device 2 is lowered into the cavity formed between the cartilage walls 10, 10' until the lower side of the body 4 rests against the upper bone surface 14. In this position, the compressed and dehydrated anchoring elements 8, 8′, 8ʺ project into the holes 12, 12′, 12ʺ in the bone surface 14, as shown in Figure 1. The compressed and dehydrated anchoring elements 8, 8′, 8ʺ are dimensioned such that they are narrower than the dimensions of the holes 12, 12', 12ʺ. The reduced dimensions and rigid, stiff properties of the compressed and dehydrated anchoring elements ensures that they can be easily and quickly inserted into the holes in the bone surface 14, without requiring undue manipulation and providing tactile feedback to the practitioner that they have been correctly inserted into the holes, which ensures that damage to the anchoring elements 8, 8′, 8ʺ and body 4 is mitigated or eliminated. Once in position, as shown in Figure 4A, the anchoring elements 8, 8′, 8ʺ are rehydrated, which causes expansion and decompression of the elements such that they substantially fill the holes 12, 12′, 12ʺ in the bone surface 14, as shown in Figure 4B. Rehydration can be caused by addition of an aqueous media such as saline, but it is preferred for rehydration to occur naturally as biological fluid (such as blood, synovial fluid, plasma, bone marrow etc.) and accompanying cells, nutrients and factors in the environment of the cartilage and bone seeps into the device 2, body 4, anchoring elements 8, 8′, 8ʺ and holes 12, 12′ and 12‴. In fact, the anchoring elements 8, 8′, 8′ are arranged to expand or rehydrate such that their dimensions are slightly larger than the dimensions of the holes 12, 12′, 12ʺ. The resilience of the hydrogel material of the anchoring elements 8, 8′, 8ʺ ensures that on complete rehydration, they resiliently grip the inner surfaces of the holes 12, 12′, 12ʺ, to secure the device 2 to the bone surface 14 and cartilage 10, 10′.

The resultant anchorage of the device in the bone surface 14 is shown in Figure 4B, where it can be seen that the anchoring elements 8, 8′, 8ʺ completely fill the holes 12, 12′, 12ʺ.

In an example of the embodiment of the device 2 described above, having two anchoring elements 8, 8", a comparison of the force required to remove the device from corresponding holes 12, 12" having a dimeter of 3.4mm, in a bone substrate of the knee of a sheep was measured for device 2 with the anchoring elements 8,8" both compressed and dehydrated to a diameter of 3.2mm, and then when the anchoring elements were rehydrated to decompress to a diameter of 4.0mm in the holes (thus being constrained by the diameter of the hole and forced to compress and resiliently grip the inner surfaces of the holes 12, 12"). The force required to remove the compressed and dehydrated anchoring elements 8,8" (and thus the device) was approximately 0.57N, while the force required to remove the decompressed anchoring elements 8,8" was approximately 28N (approximately a 49-fold increase in the force required).

Turning now to Figure 3, a second embodiment of a device 102 made by the method of the invention is shown. The device 102 includes a body 104 formed from a silk fibroin hydrogel. The body 104 includes a number of anchoring elements 108, 108', 108" projecting from the bottom surface of the body 104, as shown in Figure 3. The device 102 shown in Figure 3 is entirely compressed and dehydrated from its original uncompressed and hydrated state. Shown in dotted lines are the original dimensions of the original body 4 and anchoring elements 6, 6', 6". Thus, it can be seen that the entire device 104 shrinks in both thickness and area compared to the uncompressed and hydrated state.

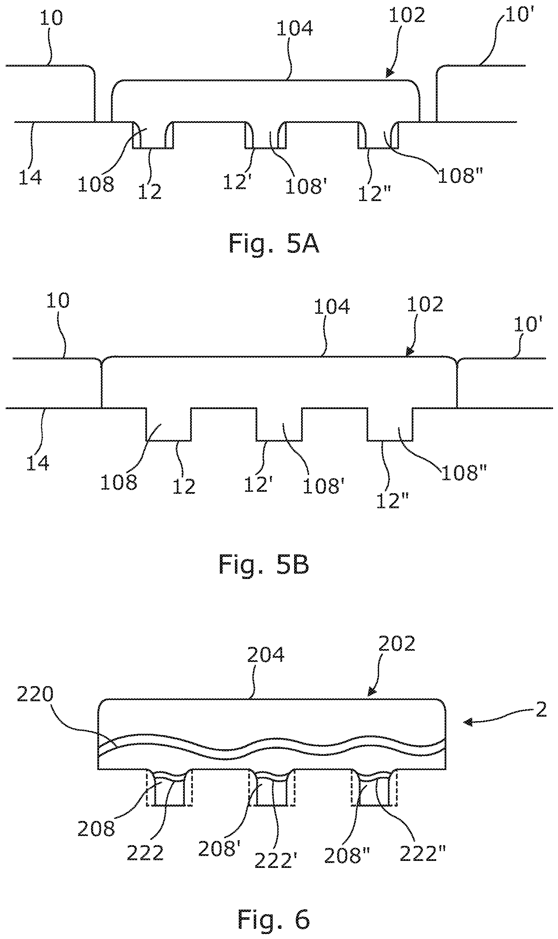

The devices 2, 102 of Figures 2 and 3 have been dehydrated via freeze-drying to preserve the shape of the body and prevent further shrinkage. This has the advantage that the position of the anchoring elements 6, 106 doesn't change when moving between the hydrated and dehydrated states, only the dimensions change. Both devices 2, 102 may be immersed in a lyo-protectant prior to and/or during freezing. Suitable lyo-protectant materials include saccharides such as trehalose and sucrose, polymers such as polyvinyl alcohol, glycerol or other polyols. Once the device or part thereof has been dehydrated it may then be further stored in a moisture free environment or immersed in an inert material such as nitrogen gas, in order to ensure that the device or part thereof does not rehydrate inadvertently before use. Use of the device 102 of Figure 3 is illustrated in Figures 5A and 5B.

Use is substantially the same as that described above for the embodiment of the device 2 of Figure 2. Figure 5A illustrates the device 102 located on the bone surface 14 within a cavity formed in articular cartilage, bounded by cartilage walls 10, 10'. The bone surface 14 has a number of holes 12, 12', 12" drilled therein, as described above in relation to Figures 4A and 4B. As can be seen from Figure 5A, the body 104 of the device 102 is dimensioned such that in the dehydrated state, the outer periphery is spaced apart from the cartilage walls 10, 10'. In addition, the anchoring element 108, 108', 108", in their dehydrated state, have a perimeter less than the perimeter and length of the holes 12, 12', 12". Upon rehydration of the device 102, both the body 104 and the anchoring elements 108, 108', 108" expand to fill both the cavity between the surfaces of the articular cartilage 10, 10' and the holes 12, 12', 12". As with the device 2 described hereinabove, the device 102 is adapted such that expansion of the body 104 and anchoring elements 108 creates a device with dimensions slightly bigger than that of the cavity formed between cartilage walls 10, 10' and holes 12, 12', 12"; and the resilience of the hydrated hydrogel forming the body 104 and anchoring elements 108 ensures that the device 102 securely grips both the cartilage walls 10, 10' and the holes 12, 12', 12".

In use of both devices 2, 102, rehydration of the dehydrated parts of the devices may be undertaken by the addition of an aqueous media such as a saline solution to the body and/or anchoring elements; but in preferred embodiments, rehydration will take place due to ingress of biological aqueous media from the surrounding tissues on which, and in which the devices 2, 102 are located; such as blood, bone marrow, interstitial fluid, synovial fluid and the like.

Figure 6 illustrates a third embodiment of a device 2 made by the method of the invention. The device 202 includes a body 204 formed of a silk fibroin hydrogel, from which extend a number of integral anchoring elements 208, 208', 208", which are also formed from the same material as the body 204. As with the devices described for Figures 2 and 3 above, the anchoring elements 208, 208', 208" are compressed and dehydrated, and thus have shrunken dimensions (periphery and volume) compared to the rehydrated anchoring elements (which are shown in dotted lines). In the embodiment of the device 204 shown in Figure 6, the body includes a fibre mesh layer 220 which extends across the entire extent of the body 204. The fibre layer 220 is formed of silk fibres and the silk fibres are woven into a mesh-like structure. The mesh-like structure includes gaps between the fibres, through which the hydrogel of the body 204 infiltrates and surrounds. In addition, the anchoring elements 208, 208', 208" also include a fibre mesh layer 222, 222', 222", respectively. In the embodiments shown in Figure 6, the fibre layers 220, 222 of the body 204 and anchoring elements 208 respectively serve to further strengthen the device 202 to enable it to withstand load pressure, and to reduce the chance of tearing or damage to the hydrogel material. The silk fibres of the fibre layers 220, 222 are also biocompatible and ensure that the device 202 has an environment conducive for infiltration of cells and other cellular material in to the device 202 after implantation. The silk fibres are also resistant to damage caused by dehydration and rehydration of the anchoring element 208.

In further embodiments of the device 202, a rigid support framework may be inserted within the body 204 before the hydrogel material is gelled, and the rigid framework may extend into the anchoring element 208, 208', 208". Such rigid frameworks ensure further strengthening of the device 202, and the rigid frameworks may be porous, to encourage larger surface area for infiltration by hydrogel material, to increase the grip of the hydrogel material around the rigid framework. Rigid supports may be formed from ceramic, polymeric, metal or alloy material, for example.

In other embodiments (not shown), the bodies of any devices may be solely compressed, rather than compressed and dehydrated, and may be retained in a compressed configuration by freezing the part(s) in the compressed configuration, or by using a retaining material such as a coating layer of biodegradable or water soluble polymer, for example, which can be applied after compression of the part(s) to keep the part in the compressed (reduced dimensions) configuration. However, in preferred embodiments the bodies and/or anchoring elements of the devices are at least dehydrated, and may be compressed then dehydrated in the compressed state.

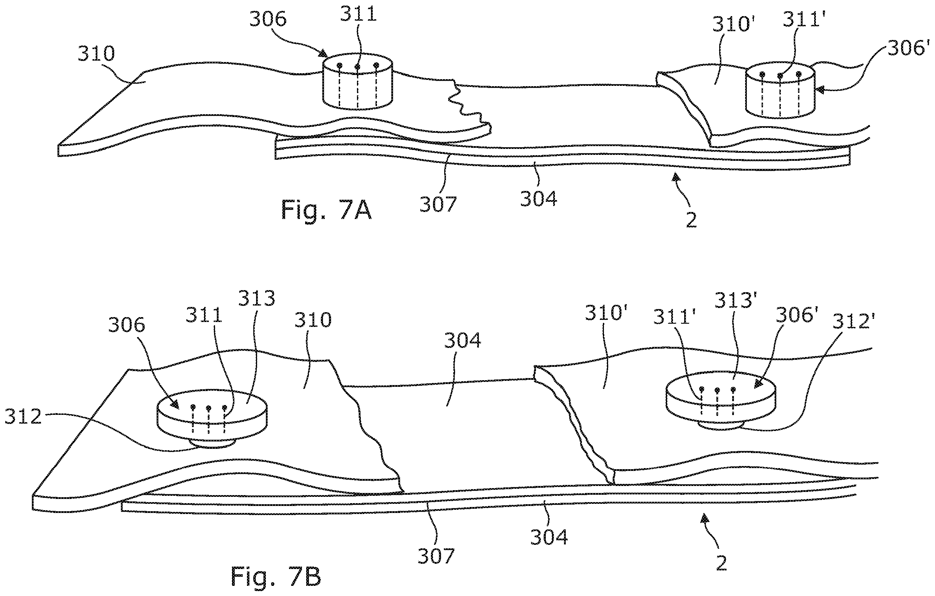

Figure 7A and 7B illustrate perspective views of a further embodiment of a device 2 made by the method of the invention, located on and spanning left 310 and right 310' sections of a torn ovine gut wall. The device 2 of Figures 7A and 7B comprises a silk fibroin body 304 which includes a silk fibre mesh layer 307 extending therethrough. The body 4 includes two anchoring elements 306,306' extending therefrom and which are located to protrude through holes drilled through the left gut wall section 310 and right gut wall section 310'. The anchoring elements include a plurality of sutures 311 and 311' extending therethrough and stitched to the silk fibre layer 307 of the body 304.

Figure 7A illustrates the device 2 positioned over the tear between the left and right gut wall sections 310,310', with compressed and freeze-dried anchoring elements 306, 306' having a smaller diameter than the uncompressed and hydrated elements. The compressed and freeze-dried elements 306, 306' have a circumference slightly less than the holes through which they are inserted, for ease of insertion through the holes.

Once the device 2 has been placed in position, as shown in Figure 7A, the anchoring elements 306,306' begin to reswell due to ingress of bodily fluids such as blood, mucus and the like. Figure 7B shows the anchoring elements 306, 306' when fully reswelled, and take the shape of a thinner shaft 312, 312' from which protrudes a disc-shaped head 313, 313' having a larger circumference than the shaft 312, 312'. It can be seen from Figure 7B that the heads 313,313' of the anchoring elements 306, 306' has a circumference greater than the shafts 312,312' and the holes within the gut wall sections 310,310', such that the body 304 of the device 2 cannot be pulled away from the sections 310,310' due to the heads 313,313' being too large to fit through the holes in the gut wall sections 310,310'.

The use of anchoring elements with a part or parts with increased dimensions (pre-compression and/or pre-drying) compared to the remainder of the anchoring elements, especially at the distal end of the anchoring elements, is particularly useful for anchoring devices made by the method of the invention into apertures or holes which either taper or extend completely through a tissue; as the part or parts can be compressed and/or dried to reduce its dimensions, then be inserted into the hole or aperture and decompressed or re-solvated to increase the dimensions back to the original dimensions, which traps the anchoring element within the hole.

In further embodiments, the structural material of the device may not be a hydrogel material, but may be a material which is solvated (either by water or another biocompatible solvent), such as a collagen sponge material or a polymeric foam material, for example.

The above embodiments are described by way of example only. Many variations are possible without departing from the scope of the invention as defined in the appended claims.

## Claims

1. A method of manufacturing an implantable tissue repair device (2) for the repair, replacement or augmentation of a biological tissue, the method comprising:
providing a device (2) comprising a biocompatible solvated structural material comprising one or more anchoring elements (6, 6′, 6ʺ); and
the method **characterised by** comprising the steps of compressing and drying at least the or each anchoring element (6, 6′, 6ʺ) to reduce one or more dimensions of at least part of the device (2).

2. A method as claimed in claim 1 comprising firstly compressing at least a part of the device (2), and subsequently drying at least the compressed part.

3. A method as claimed in claim 1 or 2 wherein the or each anchoring element (6, 6', 6") is compressed and then dried.

4. A method as claimed in any preceding claim wherein the part or parts of the device (2) which are compressed and dried are shrunk to no more than 95 % of the part's or parts' original dimensions.

5. A method as claimed in any preceding claim wherein the part or parts of the device (2) are compressed and frozen.

6. A method as claimed in any preceding claim wherein drying of the part or whole of the device (2) comprises freeze-drying the part or whole of the device (2).

7. A method as claimed in claim 5 or 6 wherein the structural material is contacted with a lyo-protectant before freezing.

8. A method as claimed in any one of claims 1 to 7 comprising compressing the solvated structural material followed by drying the compressed solvated structural material.

9. A method as claimed in any one of claims 1 to 8 wherein the structural material comprises a material selected from silk fibroin, fibrin, fibronectin, cellulose, alginate, hyaluronic acid, gelatin, chitin, chitosan and collagen.

10. A method as claimed in any one of claims 1 to 8 comprising locating a network or layer of fibres at least partially within the structural material before or during solvation of the material.

## Patentansprüche

1. Verfahren zur Herstellung einer implantierbaren Gewebereparaturvorrichtung (2) für die Reparatur, den Ersatz oder die Vermehrung eines biologischen Gewebes, wobei das Verfahren umfasst:
die Bereitstellung einer Vorrichtung, die ein biokompatibles solvatisiertes Strukturmaterial mit einem oder mehreren Verankerungselementen (6, 6', 6") aufweist, und
wobei das Verfahren **dadurch gekennzeichnet ist, dass** es die Schritte des Komprimierens und Trocknen zumindest des oder jedes Verankerungselementen (6, 6', 6") umfasst, um eine oder mehrere Dimensionen wenigstens eines Teils der Vorrichtung zu reduzieren.

2. Verfahren nach Anspruch 1, bei dem zunächst mindestens ein Teil der Vorrichtung (2) komprimiert wird und anschließend mindestens der komprimierte Teil getrocknet wird.

3. Verfahren nach Anspruch 1 oder 2, wobei mindestens das oder jedes Verankerungselement (6, 6', 6") getrocknet wird.

4. Verfahren nach einem der vorhergehenden Ansprüche, wobei der oder die Teile der Vorrichtung (2), die komprimiert und/oder getrocknet werden, auf höchstens 95 % der ursprünglichen Abmessungen des oder der Teile geschrumpft werden.

5. Verfahren nach einem der vorhergehenden Ansprüche, wobei das Teil oder die Teile der Vorrichtung komprimiert und gefroren werden.

6. Verfahren nach einem der vorhergehenden Ansprüche, wobei das Trocknen des Teils oder der Gesamtheit der Vorrichtung (2) das Gefriertrocknen des Teils oder der Gesamtheit der Vorrichtung (2) umfasst.

7. Verfahren nach Anspruch 5 oder 6, wobei das Strukturmaterial vor dem Gefrieren mit einem Lyo-Schutzmittel in Kontakt gebracht wird.

8. Verfahren nach einem der Ansprüche 1 bis 7, umfassend das Komprimieren des solvatisierten Strukturmaterials und das anschließende Trocknen des komprimierten solvatisierten Strukturmaterials.

9. Verfahren nach einem der Ansprüche 1 bis 8, wobei das Strukturmaterial ein Material umfasst, das aus Seidenfibroin, Fibrin, Fibronektin, Cellulose, Alginat, Hyaluronsäure, Gelatine, Chitin, Chitosan und Kollagen ausgewählt ist.

10. Verfahren nach einem der Ansprüche 1 bis 8, bei dem vor oder während der Solvatisierung des Materials ein Netzwerk oder eine Schicht von Fasern zumindest teilweise innerhalb des Strukturmaterials angeordnet wird.

## Revendications

1. Procédé de fabrication d'un dispositif de réparation de tissu implantable (2) pour la réparation, le remplacement ou l'augmentation d'un tissu biologique, le procédé comprenant :
prévoir un dispositif (2) comprenant un matériau structurel solvaté biocompatible comprenant un ou plusieurs éléments d'ancrage (6, 6', 6") ; et
le procédé **caractérisé en ce qu'**il comprend les étapes de comprimer et de sécher au moins le ou chaque élément d'ancrage (6, 6', 6") pour réduire une ou plusieurs dimensions d'une partie au moins du dispositif (2).

2. Procédé comme revendiqué dans la revendication 1 comprenant premièrement comprimer une partie au moins du dispositif (2) et ensuite sécher au moins la partie comprimée.

3. Procédé comme revendiqué dans la revendication 1 ou 2 dans lequel le ou chaque élément d'ancrage (6, 6', 6") est comprimé puis séché.

4. Procédé comme revendiqué dans l'une quelconque des revendications précédentes dans lequel la partie ou les parties du dispositif (2) qui sont comprimées et séchées sont rétractées à pas plus de 95 % des dimensions d'origine de la partie ou des parties.

5. Procédé comme revendiqué dans l'une quelconque des revendications précédentes dans lequel la partie ou les parties du dispositif (2) sont comprimées et congelées.

6. Procédé comme revendiqué dans l'une quelconque des revendications précédentes dans lequel sécher la partie ou l'intégralité du dispositif (2) comprend lyophiliser la partie ou l'intégralité du dispositif (2).

7. Procédé comme revendiqué dans la revendication 5 ou 6 dans lequel le matériau structurel est mis en contact avec un lyoprotecteur avant la congélation.

8. Procédé comme revendiqué dans l'une quelconque des revendications 1 à 7 comprenant l'étape de comprimer le matériau structurel solvaté, suivie de l'étape de sécher le matériau structurel solvaté comprimé.

9. Procédé comme revendiqué dans l'une quelconque des revendications 1 à 8 dans lequel le matériau structurel comprend un matériau sélectionné parmi la fibroïne de soie, la fibrine, la fibronectine, la cellulose, l'alginate, l'acide hyaluronique, la gélatine, la chitine, la chitosane et le collagène.

10. Procédé comme revendiqué dans l'une quelconque des revendications 1 à 8 comprenant placer un réseau ou une couche de fibres au moins partiellement à l'intérieur du matériau structurel avant ou pendant la solvatation du matériau.
